# EUROPÄISCHE PATENTSCHRIFT

(11) **EP 0 601 036 B1**
(45) Veröffentlichungstag und Bekanntmachung des Hinweises auf die Patenterteilung: **28.04.1999**
(21) Anmeldenummer: 92918600.5
(22) Anmeldetag: 28.08.1992
(51) Int. Cl.: A61M 15/00

(54) **MEDIZINISCHES GERÄT ZUR INHALATION VON DOSIER-AEROSOLEN**
MEDICAL DEVICE FOR INHALATING DOSES OF SPRAY
APPAREIL MEDICAL POUR INHALER DES DOSES D'AEROSOLS

(30) Priorität: 29.08.1991 DE 4128666; 28.10.1991 DE 9113361 U; 20.02.1992 DE 9202198 U; 09.04.1992 DE 9204938 U
(43) Veröffentlichungstag der Anmeldung: 15.06.1994
(73) Patentinhaber: Broncho-Air Medizintechnik AG, 73760 Ostfildern (DE)
(72) Erfinder: KLEIN, Christoph, W-5205 St. Augustin (DE)
(86) Internationale Anmeldenummer: DE9200723
(87) Internationale Veröffentlichungsnummer: WO9304718

(56) Entgegenhaltungen:
- EP-A- 0 009 667
- EP-A- 0 308 524
- DE-U- 9 113 361
- DE-U- 9 202 198
- FR-A- 1 378 213
- US-A- 3 980 074

## Beschreibung

Die Erfindung betrifft eine medizinisches Gerät zum Inhalieren von Dosier-Aerosolen gemäß dem Oberbegriff von Anspruch 1. Sie befaßt sich somit insbesondere mit einem medizinischen Gerät, bei dem die Abgabe des Aerosols und das Inhalieren gleichzeitig oder unter Zwischenschaltung einer Zwischenkammer erfolgen, in die das Medikament zuerst eingesprüht wird, um anschließend aus dieser Kammer inhaliert zu werden. Herkömmliche Aerosolbehälter haben häufig eine in den Aerosolbehälter eindrückbare Düsennadel, um das Medikament zum Inhalieren möglichst fein zerstäubt auszusprühen.

Das Inhaliergerät gemäß der zur Gattungsbildung herangezogenen EP 0009667 A1 hat ein Gehäuse mit einer den Aerosolbehälter ausnehmenden Aufnahmekammer, an die sich koaxial eine Verwirbelungskammer und ein Mundstuck anschließen. In die Verwirbelungskammer ragt relativ weit ein Abstützelement, das eine zentrale Stufenbohrung aufweist, in deren den größeren Bohrungsdurchmesser aufweisenden Abschnitt die Düsennadel des in die Aufnahmekammer eingesetzten Aerosolbehälters einschiebbar ist. In die Verwirbelungskammer münden mit relativ großem Abstand von der Stufenbohrung Lufteinlaßöffnungen, durch die die Atemluft beim Einatmen in die Verwirbelungskammer eingesaugt wird, um in der Verwirbelungskammer befindliches Medikament durch das Mundstuck in die Lunge zu fördern. Im Bereich des Mundstücks sind voneinander getrennte Einlaß- und Auslaßventile angeordnet, die einerseits ein Einatmen durch die Verwirbelungskammer und andererseits ein von der Verwirbelungskammer unabhängiges Ausatmen ermöglichen. Die in die Verwirbelungskammer mündenden Lufteinlaßöffnungen sind relativ klein dimensioniert und infolge ihrer räumlichen Lage ungeeignet, einen wesentlichen Einfluß bei der Zerstäubung bzw. Verwirbelung des Medikamentes auszuüben. Das Gerät hat eine relativ große Länge, die etwa dem Dreifachen der Höhe eines üblichen Aerosolbehälters entspricht, so daß ein ständiges Mitführen dieses Gerätes als Hand- oder Taschengerät in der Regel nicht möglich ist.

Bei einem Inhaliergerät gemäß der US-PS 48 52 561 sind das die Aufnahmekammer für den Aerosolbehälter bildende Gehäuse und das Mundstück koaxial hintereinander angeordnet, wobei der Öffnungsmechanismus für das Dosierventil des Aerosolbehälters an dem dem Mundstück entgegengesetzten Ende des Inhaliergerätes angeordnet ist. Die Aufnahmekammer für den Aerosolbehälter bildet eine Verwirbelungskammer für das Medikament, in die relativ klein dimensionierte Lufteinlaßöffnungen einmünden. Beim Öffnen des Dosierventils des Aerosolbehälters muß das mit dem Treibmittel austretende Medikament in einem Winkel von etwa 90° umgelenkt werden, so daß das Dosierventil vorzeitig verstopfen kann.

EP-0308524 A1 offenbart ein Inhaliergerät zum Zerstäuben eines Fluids, das von einer externen Quelle mit Zuführeinrichtung zugeführt wird. Dabei nimmt ein Einlaß ein Fluid von der Zufuhreinrichtung zum Auslassen des Fluids durch einen äußeren Umfang der Vorrichtung in einer Richtung stromabwärts auf. Ein Auslaß ist stromabwärts von dem Einlaß vorgesehen, um dem Fluid den Austritt zu erlauben. Zwischen dem Einlaß und dem Auslaß ist ein Verdrängungskörper angeordnet. Des weiteren ist eine Einrichtung zum Definieren mindestens eines inneren Durchgangs zu dem äußeren Durchmesser und extern und stromabwärts von dem Auslaß zum Durchlassen eines zweiten Fluids vorgesehen.

Bei der Benutzung der bekannten Inhaliergeräte besteht aufgrund der relativ klein dimensionierten, in die Verwirbelungskammer mündenden Lufteinlaßöffnungen die Gefahr der sogenannten "Atmungsangst", die noch verstärkt wird, wenn dem Einstmungemundstück Ventilelemente vorgeschaltet sind, die für ein freies Durchatmen störend sind und ein zusätzliches Hindernis bilden, an dem sich Medikament festsetzen kann.

Der Erfindung liegt die Aufgabe zugrunde, ein medizinisches Gerät zum Inhalieren von Dosier-Aerosolen so zu gestalten, daß bei der Bonutzung des Gerätes eine gegenüber den bekannten Geräten verbesserte Zerstäubung und Verwirbelung das aus dem Aerosolbehälter austratenden Medikamentes mittels der durch das Gerät eingestmeten Luft erfolgen soll, um zu erreichen, daß ein möglichst hoher Medikamentenanteil in den Bronchialbereich eintreten kann, wobei weiterhin die häufig auftretende Atmungsangst weitgehend ausgeschaltet werden soll.

Diese Aufgabe wird mit einem medizinischen Gerät gemäß dem Patentanspruch 1 gelöst.

Vorteilhafte Ausgsataltungen des Gerätes sind in den Unteransprüchen angegeben.

Der Atmungsangst wird durch entsprechend groß dimensionierte Lufthauptkanäle und eine damit ausreichende Luftzufuhr entgegengewirkt. Ein optimales Inhalieren eines großen Medikamentenanteile wird über den Zwischenweg einer Zerstäubungs- und Verwirbelungskammer gewährleistet, der ein Teil der Luft durch die Luftzweigkanäle vorzugsweise in unmittelbarer Nähe der Stufenbohrung und damit des Dosierventils des Aerosolbehälters zugeführt wird, wodurch eine zusätzliche Zerkleinerung des Medikamentenanteils auf die sogenannte lungen- oder bronchiengängige Fraktion von 3 µm und kleiner stattfindet.

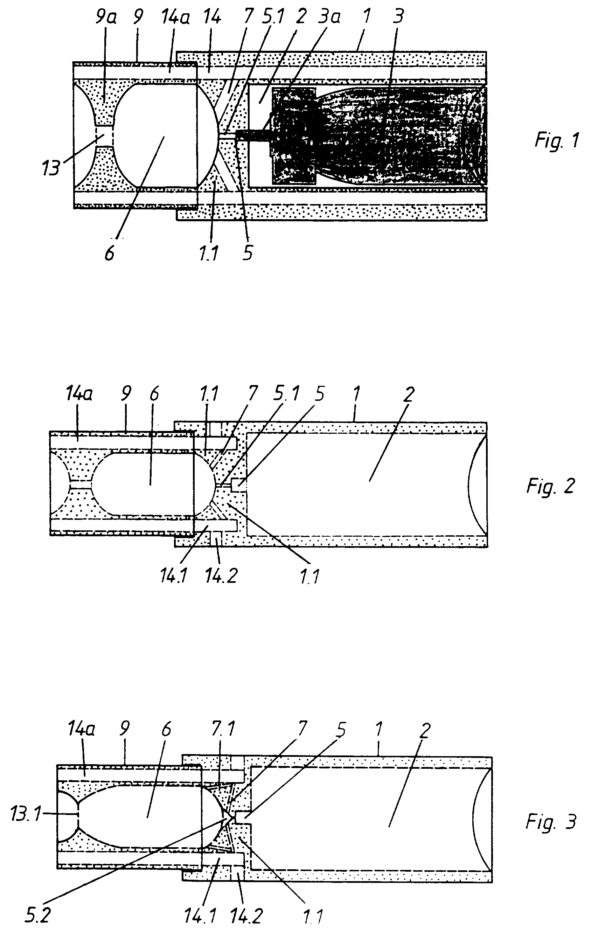

In den Figuren 1 - 5 sind verschiedene Ausführungsformen des erfindungsgemäßen Inhaliergerätes dargestellt.

Das in Figur 1 dargestellte Gerät umfaßt ein mit einem Mundstück 9 axial zusammensetzbares Gehäuse mit einer Aufnahmekammer 2, die einseitig von einer Zwischenwand 1.1 begrenzt ist, die eine Stufenbohrung 5 aufweist. Die Kammer 2 dient zur Aufnahme eines Aerosolbehälters 3, dessen Düsennadel 3a in den den größeren Durchmesser aufweisenden Abschnitt der Stufenbohrung 5 eingeschoben wird. Die Aufnahmekammer 2 ist von axialverlaufenden ersten Lufthauptkanälen 14 umgeben, die insgesamt einen großen Öffnungsquerschnitt aufweisen. An den den kleineren Durchmesser aufweisenden Abschnitt 5.1 der Stufenbohrung 5 schließt eine Verwirbelungskammer 6 an, in die mit den Lufthauptkanälen 14 in Verbindung stehende und durch die Zwischenwand 1.1 geführte Luftzweigkanäle 7, vorzugsweise in Düsennähe, in die Verwirbelungskammer 6 münden.

Das Mundstück 9 hat einen einen Teil der Verwirbelungskammer 6 bildenden Zylinderabschnitt. In Bereich des Mundstücks 9 ist die Verwirbelungskammer 6 durch eine eine zentrale Öffnung 13 aufweisende Wand 9a begrenzt und mit an die ersten Lufthauptkanäle 14 anschließenden zweiten Lufthauptkanälen 14a versehen. Zur Bildung der Lufthauptkanäle 14 und 14a sind das Gehäuse 1 und das Mundstück 9 doppelwandig mit zwischen den Zylinderwänden liegenden Stegen ausgebildet.

Das Mundstück 9 ist mit dem Gehäuse 1 beispielsweise mittels Gewinde verbindbar. Das Mundstück 9 kann an dem äußeren Ende in eine mundgerechte, z.B. ovale Form, übergehen.

Die Wände der Verwirbelungskammer 6 sind vorzugsweise poliert. Die Luftzweigkanäle 7 sind schräggestellt und der Stufenbohrung 5, 5.1 so zugeordnet, daß in der Verwirbelungskammer 6 eine optimale Zerstäubung und Verwirbelung des aus dem Behälter 3 austretenden Aerosols stattfindet.

Bei der Ausführungsform gemäß Figur 2 (siehe die die Teil-Priorität vom 28.10.1991 begründende DE-GM 9113631.0, veröffentlicht am 30.1.1992) sind an der dem Mundstück 9 zugewandten Stirnseite des Gehäuses 1 offene, relativ kurze, axial verlaufende Lufthauptkanäle 14.1 vorgesehen, in die seitliche, von der äußeren Mantelfläche des Gehäuses 1 ausgehende Anschlußanäle 14.2 münden. Die Luft zweigkanäle 7 sind an die Kanäle 14.1 angeschlossen und münden in die Verwirbelungskammer 6.

Gemäß Figur 3 (siehe die die Teilpriorität vom 20.2.1992 begründende DE-GM 9202198.0, veröffentlicht am 23.7.1992) erweitert sich der den kleineren Durchmesser der Stufenbohrung 5 aufweisende Abschnitt zu einer düsenförmigen bzw. konischen, in die Verwirbelungskammer 6 mündenden Aerosal-Auslaßöffnung 5.2, deren Öffnungswinkel zwischen 60° bis 120°, vorzugsweise bei etwa 90°, liegt, wobei die Luft-Zweigkanäle 7 in diese Auslaßöffnung 5.2 münden.

Zusätzliche Luft-Zweigkanäle 7.1, die ebenfalls von den Lufthauptkanälen 14.1 abzweigen, sind so in die Verwirbelungskammer 6 gerichtet, daß eine entlang den Wänden der Verwirbelungskammer 6 gerichtete Mantelströmung erzeugt wird.

Die zentrale, an die Verwirbelungskammer 6 anschließende Öffnung 13.1 hat einen relativ großen Querschnitt.

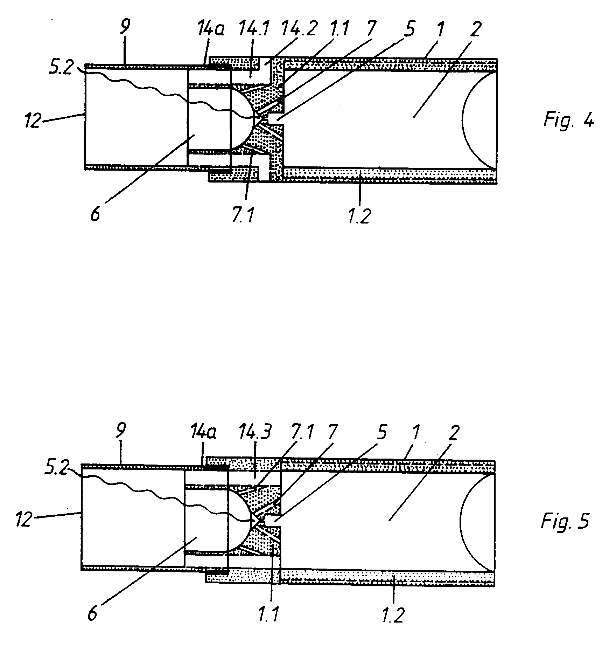

Gemäß Figur 4 (siehe die die Teilpriorität vom 9. April 1992 begründende, nicht veröffentlichte DE-GM 9204938.9) ist das Gehäuse 1 abweichend von Figur 3 an der Innenwand mit axial verlaufenden Abstandsstegen 1.2 versehen, so daß nach dem Einschieben eines Aerosol-Behälters in die Aufnahmekammer 2 zwischen dem Aerosol-Behälter und der Innenwand axial verlaufende Luftkanäle freibleiben. An die Aufnahmekammer 2 anschließende Luft-Zweigkanäle 7 münden in die sich düsenertig erweiternde Aerosol-Auslaßöffnung 5.2.

Das Mundstück 9 besteht aus einem Zylinderrohr, dessen Innenraum einen Teil der Verwirbelungskammer 6 bildet, der im wesentlichen über seine gesamte Länge einen gleichförmigen zylindrischen Querschnitt hat, so daß die Mundstücksöffnung 12 den gleichen Querschnitt hat, wie der davor liegende Innenraum des Mundstücks 9.

Die Lufthauptkanäle 14.1 und die Anschlußkanäle 14.2, sowie die in die Verwirbelungskammer 6 mündenden Luft-Zweigkanälan 7.1 entsprechen im wesentlichen der Ausführungsform gemäß Figur 3. Bei der Ausführungsform gemäß Figur 4 hat der Innenraum des Mundstücks 9 einen Durchmesser von 12 - 18 mm, vorzugsweise 15 mm, wobei die Lufthauptkanäle 14a relativ kurz sind, so daß die daraus austretende Luft eine entlang der Mundstückinnenwand strömende Mantelströmung bildet.

Bei der Ausführungsform gemäß Figur 5 (ohne Priorität) schließen sich abweichend von Figur 4 durch das mit Abstandsstegen 1.2 versehene Gehäuse 1 geführte Lufthauptkanäle 14.3 axial ausgerichtet unmittelbar an die Aufnahmekammer 2 an, so daß die z.B. gemäß Figur 4 seitlich austretenden Anschlußkanäle 14.2 entfallen.

Das Gehäuse 1 und das Mundstück 9 bestehen vorzugsweise aus V2A- oder V4A-Stahl, Aluminium oder Kunststoff.

Durch die geradlinige Anordnung des Gerätes wird insbesondere auch die Anwendung im Liegen, z.B. in der Nacht oder bei bettlägrigen Patienten erleichtert und optimiert.

Zur Steuerung der durch die zweiten Lufthauptkanäle 14a strömenden Luftmenge einerseits und der durch Luftzweigkanäle 7; 7.1 strömenden Luftmenge andererseits, ist das Mundstück 7 in verschiedenen Drehstellungen fixiert mit dem Gehäuse (1) verschraubbar.

## Patentansprüche

1. Medizinisches Gerät zum Inhalieren von Dosier-Aerosolen enthaltend ein zylindrisches Gehäuse (1) mit einer Aufnahmekammer (2) für einen Aerosolbehälter (3), eine Zerstäubungs- und Verwirbelungskammer (6) und ein an das Gehäuse koaxial anschließendes Mundstück (9),
dadurch gekennzeichnet,
daß zwischen der Aufnahmekammer (2) und der Verwirbelungskammer (6) eine Zwischenwand (1.1) vorgesehen ist, die mit einer Stufenbohrung (5) versehen ist, deren den größeren Durchmesser aufweisender Bohrungsabschnitt an die Aufnahmekammer (2) und deren den kleineren Durchmesser aufweisender Bohrungsabschnitt als Aerosol-Auslaßöffnung (5.2) an die Verwirbelungskammer (6) anschließt, wobei die Zerstäubungs- und Verwirbelungskammer (6) gemeinsam von dem Mundstück (9) und dem Gehäuse (1) gebildet ist und wobei ferner das Gehäuse axial verlaufende erste Lufthauptkanäle aufweist, an die sich zweite Lufthauptkanäle (14a) des Mundstücks anschließen und an die Luftzweigkanäle (7) angeschlossen sind, die schräg zur Zwischenwand (1.1) in dieser verlaufen und die Stufenbohrung (5) umgebend in die Verwirbelungskammer münden.

2. Medizinisches Gerät nach Anspruch 1,
dadurch gekennzeichnet,
daß das Gehäuse (1) mehrere erste an der dem Mundstück (9) zugewandten Stirnseite des Gehäuses (1) offene Lufthauptkanäle (14.1) aufweist, in die seitliche Anschlußkanäle (14.2) münden.

3. Medizinisches Gerät nach Anspruch 1,
dadurch gekennzeichnet,
daß die ersten Lufthauptkanäle (14.3) die Aufnahmekammer (2) mit der Zerstäubungs- und Verwirbelungskammer (6) verbinden.

4. Medizinisches Gerät nach Anspruch 2 oder 3,
dadurch gekennzeichnet,
daß die Zwischenwand (1.1) weitere die Lufthauptkanäle (14; 14.1; 14.3) mit der Verwirbelungskammer (6) verbindende Kanäle (7.1) aufweist, die die Luftzweigkanäle (7) umgebend in die Verwirbelungskammer (6) münden.

5. Medizinisches Gerät nach einem der Ansprüche 1 bis 4,
dadurch gekennzeichnet,
daß das Mundstück (9) einen zur Benutzerseite offenen Zylinderabschnitt aufweist, dessen Öffnungsquerschnitt im wesentlichen dem Innenquerschnitt des durch das Mundstück (9) gebildeten Abschnittes der Verwirbelungskammer (8) entspricht.

6. Medizinisches Gerät nach Anspruch 1,
dadurch gekennzeichnet,
daß das Gehäuse (1) an seiner Innenwand axial verlaufende Abstandsstege (1.2) aufweist.

7. Medizinisches Gerät nach Anspruch 1,
dadurch gekennzeichnet,
daß das Gehäuse (1) und das Mundstück (9) miteinander verschraubbar sind.

8. Medizinisches Gerät nach Anspruch 1,
dadurch gekennzeichnet,
daß die Aerosol-Auslaßöffnung (5.2) einen sich zur Verwirbelungskammer (6) düsenartig konisch erweiternden Querschnitt mit einem Öffnungswinkel zwischen 60° bis 120°, vorzugsweise etwa 90°, hat.

9. Medizinisches Gerät nach Anspruch 8,
dadurch gekennzeichnet,
daß die Luft-Zweigkanäle (7) in den sich konisch erweiternden Abschnitt der Aerosol-Auslaßöffnung (5.2) münden.

10. Medizinisches Gerät nach Anspruch 1 oder 4,
dadurch gekennzeichnet,
daß die Luft-Zweigkanäle (7, 7.1) strahlenförmig in die Verwirbelungskammer (6) münden.

11. Medizinisches Gerät nach Anspruch 1,
dadurch gekennzeichnet,
daß zur Steuerung der durch die zweiten Lufthauptkanäle (14a) strömenden Luftmenge einerseits und der durch Luftzweigkanäle (7; 7.1) strömenden Luftmenge andererseits das Mundstück (7) in verschiedenen Drehstellungen mit dem Gehäuse (1) verschraubbar ist.

12. Medizinisches Gerät nach Anspruch 1,
dadurch gekennzeichnet,
daß die ersten Lufthauptkanäle (14) durchlaufend durch das Gehäuse (1) hindurchgeführt sind.

## Claims

1. Medical device for inhaling metered dosage aerosols comprising a cylindrical casing (1) with a receiving chamber (2) for an aerosol container (3), a nebulising and dispersing chamber (6) and a mouthpiece (9) connecting coaxially to the casing, characterised in that between the receiving chamber (2) and the dispersing chamber (6) an intermediate wall (1.1) is provided which is fitted with a stepped hole (5), of which the hole section having the larger diameter is connected to the receiving chamber (2) and the hole section having the smaller diameter is connected as aerosol outlet (5.2) to the dispersing chamber (6), whereby the nebulising and dispersing chamber (6) is formed jointly by the mouthpiece (9) and the casing (1), and whereby the casing additionally has axially extending first main air ducts, to which second main air ducts (14a) of the mouthpiece connect and to which branch air ducts (7) are connected, which run in the intermediate wall (1.1) on an incline thereto and, surrounding the stepped hole (5), feed into the dispersing chamber.

2. Medical device according to Claim 1, characterised in that the casing (1) has several first main air ducts (14.1), which are open on the face side of the casing (1) facing the mouthpiece (9) and into which lateral connection ducts (14.2) feed.

3. Medical device according to Claim 1, characterised in that the first main air ducts (14.3) connect the receiving chamber (2) to the nebulising and dispersing chamber (6).

4. Medical device according to Claim 2 or 3, characterised in that the intermediate wall (1.1) has further ducts (7.1) connecting the main air ducts (14; 14.1; 14.3) with the dispersing chamber (6) which, surrounding the branch air ducts (7), feed into the dispersing chamber (6).

5. Medical device according to one of Claims 1 to 4, characterised in that the mouthpiece (9) has a cylinder section open to the user side, the opening cross-section of which essentially corresponds to the inner cross-section of the section of the dispersing chamber (6) formed by the mouthpiece (9).

6. Medical device according to Claim 1, characterised in that the casing (1) has spacer webs (1.2) extending axially on its inside wall.

7. Medical device according to Claim 1, characterised in that the casing (1) and the mouthpiece (9) may be screwed together.

8. Medical device according to Claim 1, characterised in that the aerosol outlet (5.2) has a cross-section, which widens conically towards the dispersing chamber (6) in the manner of a nozzle, with a opening angle of between 60° and 120°, preferably of about 90°.

9. Medical device according to Claim 8, characterised in that the branch air ducts (7) feed into the conically widening section of the aerosol outlet (5.2).

10. Medical device according to Claim 1 or 4, characterised in that the branch air ducts (7, 7.1) feed radially into the dispersing chamber (6).

11. Medical device according to Claim 1, characterised in that the mouthpiece (7) may be screwed to the casing (1) in various rotational positions to control, on the one hand, the amount of air flowing through the second main air ducts (14a) and, on the other hand, the amount of air flowing through branch air ducts (7; 7.1).

12. Medical device according to Claim 1, characterised in that the first main air ducts (14) are continuously passed through the casing (1).

## Revendications

1. Appareil médical pour l'inhalation d'aérosol en dose, comprenant un corps (1) cylindrique avec une chambre de réception (2) pour un réservoir d'aérosol (3), une chambre de pulvérisation et de turbulence (6) et un embout buccal (9) se raccordant coaxialement au corps,
***caractérisé par le fait***
qu'entre la chambre de réception (2) et la chambre de turbulence (6) est disposée une cloison (1.1) qui est munie d'un trou étagé (5) dont le tronçon de plus grand diamètre se raccorde à la chambre de réception (2) et dont le tronçon de plus petit diamètre se raccorde en tant qu'ouverture de sortie d'aérosol (5.2) à la chambre de turbulence (6), la chambre de pulvérisation et de turbulence (6) étant délimitée conjointement par l'embout buccal (9) et le corps (1) et le corps présentant, en outre, des premiers canaux principaux d'air s'étendant axialement, auxquels se raccordent des deuxièmes canaux principaux d'air (14a) de l'embout buccal et auxquels sont raccordés des canaux secondaires d'air (7) qui s'étendent de façon oblique par rapport à la cloison (1.1) dans cette dernière et débouchent dans la chambre de turbulence en entourant le trou étagé (5).

2. Appareil médical suivant la revendication 1,
***caractérisé par le fait***
que le corps (1) présente plusieurs premiers canaux principaux d'air (14.1) qui sont ouverts sur le côté frontal du corps (1) tourné vers l'embout buccal (9) et dans lesquels débouchent des canaux latéraux de raccordement (14.2).

3. Appareil médical suivant la revendication 1,
***caractérisé par le fait***
que les premiers canaux principaux d'air (14.3) relient la chambre de réception (2) à la chambre de pulvérisation et de turbulence (6).

4. Appareil médical suivant la revendication 2 ou 3,
***caractérisé par le fait***
que la cloison (1.1) présente d'autres canaux (7.1) qui relient les canaux principaux d'air (14; 14.1; 14.3) à la chambre de turbulence (6) et qui débouchent dans la chambre de turbulence (6) en entourant les canaux secondaires d'air (7).

5. Appareil médical suivant l'une des revendications 1 à 4,
***caractérisé par le fait***
que l'embout buccal (9) présente un tronçon de cylindre ouvert vers le côté utilisateur, dont la section d'ouverture correspond essentiellement à la section intérieure du tronçon de la chambre de turbulence (6) constitué par l'embout buccal (9).

6. Appareil médical suivant la revendication 1,
***caractérisé par le fait***
que le corps (1) présente sur sa paroi intérieure des nervures d'espacement (1.2) s'étendant axialement.

7. Appareil médical suivant la revendication 1,
***caractérisé par le fait***
que le corps (1) et l'embout buccal (7) peuvent être reliés l'un à l'autre par vissage.

8. Appareil médical suivant la revendication 1,
***caractérisé par le fait***
que l'ouverture de sortie d'aérosol (5.2) présente une section augmentant en cône, à la manière d'une tuyère, vers la chambre de turbulence (6) avec un angle d'ouverture compris entre 60° et 120°, de préférence un angle d'ouverture d'environ 90°.

9. Appareil médical suivant la revendication 8,
***caractérisé par le fait***
que les canaux secondaires d'air (7) débouchent dans le tronçon de l'ouverture de sortie d'aérosol (5.2) dont la section augmente en cône.

10. Appareil médical suivant la revendication 1 ou 4,
***caractérisé par le fait***
que les canaux secondaires d'air (7, 7.1) débouchent en forme de rayons dans la chambre de turbulence (6).

11. Appareil médical suivant la revendication 1,
***caractérisé par le fait***
qu'en vue de la commande du débit d'air traversant les deuxièmes canaux principaux d'air (14a), d'une part, et du débit d'air traversant les canaux secondaires d'air (7; 7.1), d'autre part, l'embout buccal (7) peut être assemblé par vis dans différentes positions angulaires avec le corps (1).

12. Appareil médical suivant la revendication 1,
***caractérisé par le fait***
que les premiers canaux principaux d'air (14) traversent le corps (1) de part en part.
